# EUROPEAN PATENT APPLICATION

(11) **EP 0 582 276 A1**
(43) Date of publication of application: **09.02.1994**
(21) Application number: 93112434.1
(22) Date of filing: 03.08.1993
(51) Int. Cl.: B21G 1/00, A61B 17/06

(54) **Needles made from non-round stock**

(30) Priority: 03.08.1992 US 924157
(71) Applicant: United States Surgical Corporation, Norwalk, Connecticut 06856 (US)
(72) Inventor: Haroldsen, Michael, Oxford, CT 06404 (US)
(74) Representative: Marsh, Roy David

(57) **Abstract**

Needles are made from wire having a non-round cross-sectional shape. The needles preferably have at least one cutting edge (A,A',A'')

## Description

### FIELD OF THE INVENTION

The present invention relates generally to methods of manufacturing surgical needles. More particularly, this invention relates to methods of manufacturing surgical needles having cutting edges starting from non-round wire.

### BACKGROUND OF THE INVENTION

Generally speaking, a surgical needle includes a point section, a body section, and a suture attachment section.

The point section of a surgical needle is normally tapered in some way to provide a point at one end of the needle, thereby facilitating penetration of the needle into tissue. Cutting edges may also be formed in the point section to further facilitate passage of the needle through tissue. In some instances, at least some portion of the point section of a cutting needle will have a non-round configuration to provide the cutting edge. Additionally, the point section may be hollow ground, that is, ground so as to have a generally concave surface (either curved or V-shaped) adjacent the cutting edge. Needles with non-round point sections are shown, for example, in U.S. Patent Nos. 5,041,127; 5,030,228; 5,002,565; 5,002,564; 4,932,961; 4,660,559; 4,524,771; 4,513,747; 4,128,351; 3,636,955; 3,265,070; 3,038,475; 2,869,550; 2,811,157; 2,516,710; 1,648,451; and 1,592,897. Hollow ground needles are described, for example, in U.S. Patent Nos. 5,030,228; 5,002,565; 5,002,564; 4,932,961; 4,660,559; and 3,038,475, the disclosures of which are incorporated herein by reference.

The body section of a surgical needle is usually where a surgeon will hold the needle during use. In many cases, the needle is held and manipulated by a needle holder. To ensure secure gripping of the needle and to better control the orientation of the needle during handling, the body section of many surgical needles includes a non-round portion at which the needle may be grabbed with a needle holder. In some instances, the entire body section is non-round in cross-section. Needles in which at least a portion of the body section is non-round are shown, for example, in U.S. Patent Nos. 5,041,127; 5,030,228; 5,002,564; 4,932,961; 4,799,484; 3,265,070; 1,648,451; and 1,110,468.

The suture attachment section is normally at the blunt end of the needle, and provides a means for securing a suture to the needle. For example, the securing means may include a bore or hole drilled in the blunt end of the needle into which the end of a suture can be inserted. As another example, the securing means may be a trough or slot formed near the blunt end of the needle and into which the end of a suture may be placed and secured by crimping. As yet another example, a reduced shank may be provided at the butt end of the needle and a connector, usually in the form of a tubular structure, placed thereover for receiving the end of a suture. These and other securing means for attaching a suture to a needle are known to those skilled in the art. See, for example, U.S. Patent Nos. 5,059,212; 5,001,323; 4,957,502; 4,700,043; and 3,892,240, the disclosures of which are incorporated herein by reference.

Prior art needles have generally been made from round wire. Any non-round sections of prior art needles have been formed in the round stock by grinding and/or pressing to produce the desired shape. Since conventionally many of the forming operations involve manual labor, each step added to the needle forming process adds considerably to the cost of manufacturing the needle.

It is therefore an object of the present invention to provide an method of manufacturing surgical needles in a more efficient manner.

### SUMMARY OF THE INVENTION

Surgical needles are manufactured in accordance with the present invention from non-round stock, such as, for example, from a coil of wire which is not round in cross-section. The non-round wire may have any cross-sectional shape, including triangular, rectangular, cross-shaped, diamond-shaped, pentagonal, hexagonal or lens shaped wire. The method of the invention comprises cutting a length of non-round wire to produce a needle blank and tapering the needle blank to form a point section at one end of the needle blank, the point section having at least one cutting edge. In a preferred embodiment, the method includes hollow grinding the point section adjacent the cutting edge.

In a particularly useful embodiment the needle blank is tapered such that the cross-sectional shape of the point section is substantially the same as the cross-sectional shape of the stock wire over a substantial portion of the point section, thereby providing a smooth transition between the point section and body section of the needle.

### BRIEF DESCRIPTION OF THE DRAWINGS

The present invention will more readily be understood by the accompanying description of the drawings and the detailed description of the invention, in which:
FIGS. 1-15 are cross-sectional views of various non-round wires useful in the present invention;
FIGS. 16A and B are schematic cross-sectional views of the point section of needle blanks having a lens shaped cross-section which have been ground in two planes, and hollow ground, respectively.
FIGS. 17A-C are schematic perspective views of the point section of needle blanks having a triangular cross-section which have been ground on one, two and three sides, respectively; and
FIGS. 18A and B are cross sectional views of two embodiments of the point section shown in FIG. 17C which are planar ground and hollow ground, respectively.

### DETAILED DESCRIPTION OF THE PREFERRED EMBODIMENTS

Cutting a length of non-round wire to form a needle blank is the first step in making surgical needles in accordance with this invention. The length of needle blank may be from one to 2.5 times the length of the final needle to be produced. Preferably, the length of the needle blank is from about one to about 1.25 times the length of the final needle to be produced. Most preferably, the needle blank is no more than about ¼ inch longer than the length of the final needle to be produced. Normally, the needle blank will be from about ⅛inch to about 4 inches long. In its broadest dimension, the width of the non-round wire may range from about 0.001 to about 0.075 inches. The non-round wire may be made of any material conventionally used in the manufacture of surgical needles such as, for example, stainless steel.

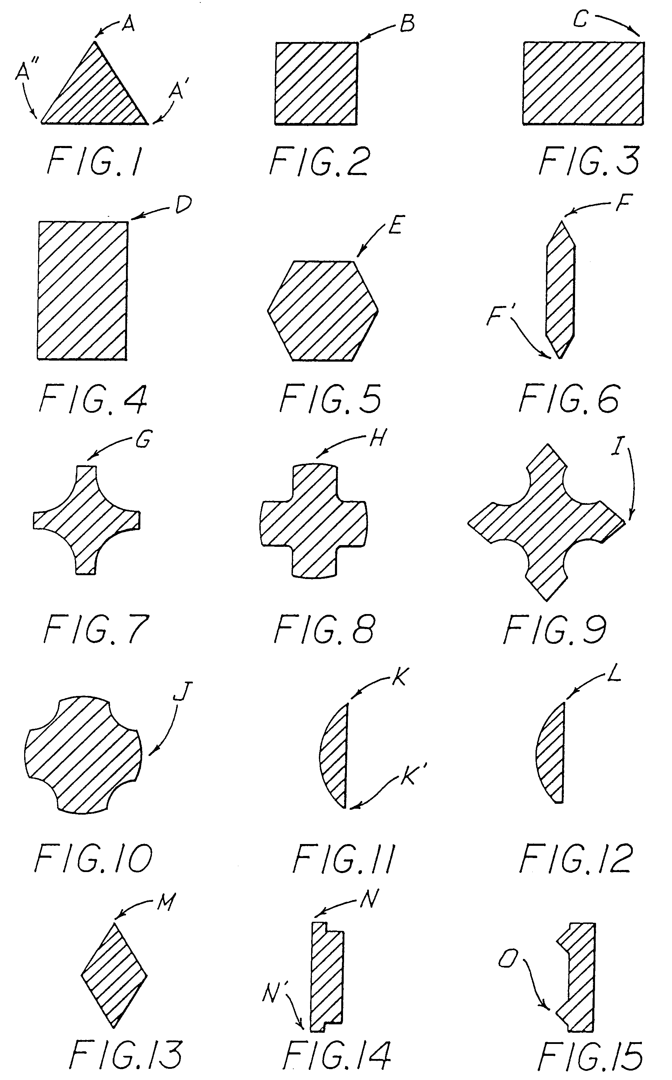

The term "non-round" wire is intended to embrace any wire that in cross-section has a shape other than round. The non-round wire may have a triangular cross-section, for example, such as that shown in FIG. 1. Wires having a rectangular cross-section, such as the square shape shown in FIG. 2 or the rectangles shown in FIGS. 3 and 4, may also be employed. Suitable hexagonal cross-sections are shown in FIGS. 5 and 6. The wire may also be generally cross-shaped in cross-section. Four variations of cross-shaped wires are shown in FIGS. 7, 8, 9 and 10. Wires which are lens shaped in cross-section, for example, such as the shapes illustrated in FIGS. 11 and 12 are also suitable. FIG. 13 shows a diamond shaped cross-sectional shape of a non-round wire. Other suitable non-round cross-sectional shapes are shown in FIGS. 14 and 15. It should be understood that FIGS. 1 to 15 are merely representative of the many non-round wire cross-sectional shapes which may be employed in the method of the present invention.

The non-round wire which is used as the starting material in the present invention can be prepared by any conventional method, such as, for example, by using a Turk's-head machine. Where the non-round wire is provided in a coiled configuration, the method of this invention may include the step of straightening the wire prior to cutting the needle blank or straightening the needle blank once it has been cut. This straightening step removes any residual curve resulting from coiling of the wire. This straightening step may also facilitate handling of the needle blank for further processing steps.

The non-round cross-sectional shape of the wire will, in particularly useful embodiments, be maintained as the non-round cross-sectional shape of the body section of the needle. It may be desirable to impart a round cross-sectional configuration to a portion of the needle such as, for example, in connection with forming the suture attachment section as discussed infra.

Once the needle blank has been cut from the non-round wire, and perhaps straightened, one end of the needle blank is tapered to form a point section at one end thereof, the point section having at least one cutting edge. Tapering can be accomplished using any known technique, provided at least one cutting edge is formed. Preferably, the cutting edge is formed at or corresponds to an edge of the non-round stock. For example, for a needle blank made from non-round wire of the configurations shown in FIGS. 1-15, a cutting edge may be formed at or corresponding to edges A through O, respectively. It is also possible that more than one edge of the non-round wire may become or correspond to a cutting edge as the needle is tapered. In FIG. 1, for example, the edges labelled A, A' and A" may each be the basis for a cutting edge. As another example, in FIG. 6, the edges labelled F and F' may each become a cutting edge. As yet other examples, in FIG. 11 and FIG. 14 the edges labelled K and K' and the edges labelled N and N', respectively, may become cutting edges. It should be understood that multiple cutting edge needles may also be formed from the other configurations shown in FIGS. 1-15.

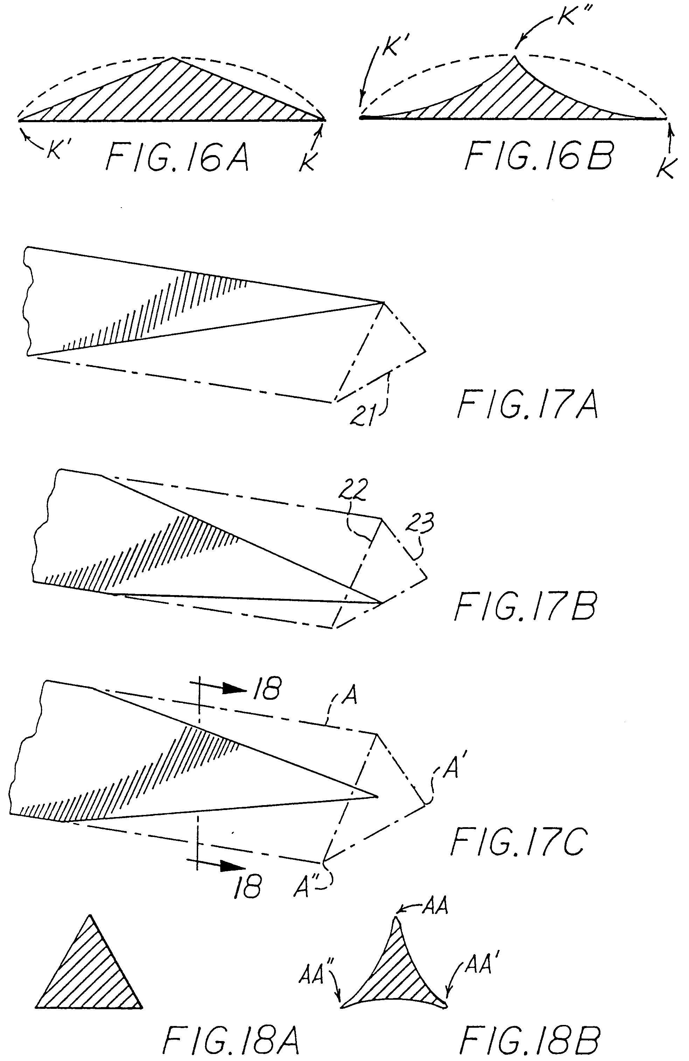

By way of illustrating the tapering to form a point section having at least one cutting edge, a needle blank having the cross-sectional shape shown in FIG. 11 may be ground into a triangular point section by grinding in two planes. This is schematically shown in FIG. 16A, wherein the original lens shaped cross-sectional shape of the needle blank is shown in phantom lines and the ground triangular shape is shown in solid lines. Edges K and K' are formed into cutting edges. FIG. 16B also shows the original lens shape of the needle blank in phantom lines, however, the point section has now been hollow ground to produce three cutting edges at K, K' and K''. The hollow grinding may be performed after the planar grinding to form the taper. Alternatively, the hollow grinding itself may form the taper.

In a particularly useful embodiment of this invention, the cross-sectional shape of the point section formed by tapering is essentially the same as the cross-sectional shape of the non-round wire, over a substantial portion of the point section. Having a point section having substantially the same cross-section as the body section will result in a smooth transition between these two sections of the needle. For example, for a needle blank made from a triangular wire, the point section would have a triangular cross-section over a substantial portion of its length. The actual point may be formed by grinding one, two or three sides of the triangular stock. These three possibilities are shown schematically in FIGS. 17A-C, wherein the needle blank prior to any grinding is shown in phantom lines and the ground point section is shown in solid lines. In FIG. 17A, only side 21 of the needle blank has been ground to produce a tapered point section having a triangular cross-section. In FIG. 17B, sides 22 and 23 have both been ground to give a tapered point section having a triangular cross-section. In FIG. 17C, all three sides of the triangular needle blank have been ground to give a tapered point section having a triangular cross-section. As mentioned previously, one or all three edges of the triangular point section may form a cutting edge.

The tapering to form a point section may be achieved by planar grinding or by hollow grinding the needle blank. For example, with respect to the embodiment shown in FIG. 17C, planar grinding will produce a point section with the cross-sectional shape shown in FIG. 18A while hollow grinding will produce a point section having the cross-sectional shape shown in FIG. 18B. Alternatively, the tapering may be achieved by planar grinding, with hollow grinding being performed as a separate, subsequent step.

A comparison of FIGS. 17C and 18B illustrates what is meant by a cutting edge "corresponding to" an edge of the needle blank. In FIG. 17C, edge A of the original needle blank does not itself form the cutting edge. However, cutting edge AA in FIG. 18B corresponds to edge A of FIG. 17C. Similarly, cutting edges AA' and AA'' in FIG. 18B correspond to edges A' and A'', respectively, of FIG. 17C.

A suture attachment section may be formed at the end of the needle blank opposite the point section. Forming the suture attachment section may be achieved by any conventional method. As discussed above, for example, the suture attachment section may be formed by drilling an axial hole or bore into one end of the needle blank such as by laser drilling. As another example, a slot or trough may be cut into an end of the needle blank for receiving the end of a suture. As yet another example, a reduced shank may be formed onto which a connecting member may be placed, the connecting member having means for receiving a suture. As part of forming the suture attachment section of the needle, it may be advantageous to round off a portion of the non-round body section of the needle.

It should be understood that the sequence of steps may be varied in accordance with this invention. For example, the forming of the suture attachment section may be performed before, during or after the tapering step. Similarly, the hollow grinding step may be performed before, during or after the tapering step. It should also be understood that other operations such as polishing, etching, plating, hardening, curving, cleaning, and/or drying may be performed on the needle blank or needles manufactured in accordance with the present invention.

## Claims

**1.** A method of making a surgical needle comprising:
cutting a length of non-round wire to produce a needle blank;
tapering the needle blank to form a point section at one end thereof; and
hollow grinding at least one surface of the point section to form at least one cutting edge.

**2.** A method as claimed in claim 1 wherein the point section has essentially the same cross-sectional shape as the non-round wire.

**3.** A method as in claim 1 or 2 wherein the cutting edge is formed from an edge of the non-round wire.

**4.** A method as in any one of the preceding claims further comprising the step of straightening the needle blank after it is cut from the non-round wire.

**5.** A method as claimed in any one of the preceding claims wherein the length of the needle blank is no more than 6mm (.25 inches) longer than the final needle produced.

**6.** A method as claimed in any one of the preceding claims further comprising the step of forming a suture attachment section on the needle blank.

**7.** A method as claimed in claim 6 wherein said step of forming a suture attachment section comprises laser drilling one end of said needle blank.

**8.** A method as claimed in claim 6 or 7 wherein said step of forming a suture attachment section comprises forming a shank of reduced dimension on one end of the needle blank.

**9** A method as claimed in claim 6, 7 or 8 wherein said step of forming a suture attachment section comprises forming a groove near one end of the needle blank.

**10.** A method as claimed in any one of claims 6 to 9 wherein said step of forming a suture attachment section is performed before said tapering step.

**11.** A method as claimed in any one of the preceding claims wherein said tapering and hollow grinding steps are simultaneous.

**12.** A method as claimed in any one of claims 1 to 10 wherein said hollow grinding step is performed after said tapering step.

**13.** A method as claimed in any one of the preceding claims wherein the non-round wire is triangular in cross-section.

**14.** A method as claimed in claim 13, wherein said tapering step comprises grinding the three sides of the needle blank simultaneously.

**15.** A method as claimed in claim 14 wherein said grinding is substantially equal on all sides.

**16.** A method as claimed in claim 13 or 14 wherein said grinding is hollow grinding.

**17.** A method as claimed in any one of the preceding claims further comprising the step of polishing the point section.

**18.** A method as claimed in any one of the preceding claims further comprising the step of rounding off at least a portion of the needle blank.

**19.** A method as claimed in claim 18 wherein the portion of the needle blank rounded off is at one end thereof.

**20.** A method as claimed in claim 18 or 19 wherein said rounding off step is performed prior to said step of forming a suture attachment section.
